(19) Europäisches Patentamt
European Patent Office
Office européen des brevets

(11) Veröffentlichungsnummer **0 033 879**
**A2**

# (12) EUROPÄISCHE PATENTANMELDUNG

(21) Anmeldenummer: 81100526.3

(22) Anmeldetag: 24.01.81

(51) Int. Cl.³: **C 07 C 133/10**

(30) Priorität: 04.02.80 DE 3003978

(43) Veröffentlichungstag der Anmeldung: 19.08.81
Patentblatt 81/33

(84) Benannte Vertragsstaaten: BE CH DE FR GB LI

(71) Anmelder: **BASF Aktiengesellschaft,**
**Carl-Bosch-Strasse 38, D-6700 Ludwigshafen (DE)**

(72) Erfinder: **Barl, Manfred, Dr., Pappelstrasse 2,**
**D-6701 Otterstadt (DE)**
Erfinder: **Horacek, Heinrich, Dr., Londoner Ring 11,**
**D-6700 Ludwigshafen (DE)**
Erfinder: **Himmele, Walter, Dr., Eichenweg 14,**
**D-6909 Walldorf (DE)**

(54) **1,3-Bis-(dialkylaminoalkyl)-guanidine und Verfahren zu ihrer Herstellung.**

(57) Neue 1,3-Bis-(dialkylaminoalkyl)-guanidine und ein neues Verfahren zu ihrer Herstellung durch Umsetzung von Guanidiniumsalzen mit Diaminen bei 140 bis 250° C.

Die nach dem Verfahren der Erfindung herstellbaren neuen 1,3-Bis-(dialkylaminoalkyl)-guanidine sind wertvolle Ausgangsstoffe für die Herstellung von Hilfsmitteln auf dem Kunststoffgebiet, Farbstoffen, Pflanzenschutzmitteln und Pharmazeutika.

EP 0 033 879 A2

# 1,3-Bis-(dialkylaminoalkyl)-guanidine und Verfahren zu ihrer Herstellung

Die Erfindung betrifft die neuen 1,3-Bis-(dialkylaminoalkyl)-guanidine und ein neues Verfahren zu ihrer Herstellung durch Umsetzung von Guanidiniumsalzen mit Diaminen bei 140 bis 250°C.

Disubstituierte symmetrische Guanidine können durch Umsetzung von Aminen mit Chlor- oder Bromcyan (US-PS 1 639 725, US-PS 2 254 009, DE-PS 19 58 095) hergestellt werden. Eine weitere Synthese solcher Guanidine ist die Entschwefelung von symmetrisch disubstituierten Thioharnstoffen mit Schwermetallsalzen, z.B. Quecksilber- oder Bleisalzen, und Umsetzung der intermediär gebildeten Carbodiimide mit Ammoniak.

Diese Verfahren haben den Nachteil, daß die Reaktionskomponenten toxisch oder schwer zugänglich sind.

Ein Zusammenschmelzen von Dicyandiamiden und primären oder sekundären Aminsalzen führt zu unsymmetrisch monosubstituierten oder disubstituierten Guanidinen (Houben-Weyl, Methoden der Organischen Chemie, Band 8, Seiten 183 bis 188; US-PS 2 230 965.

Es wurde nun gefunden, daß man 1,3-Bis-(dialkylaminoalkyl)-guanidine der Formel

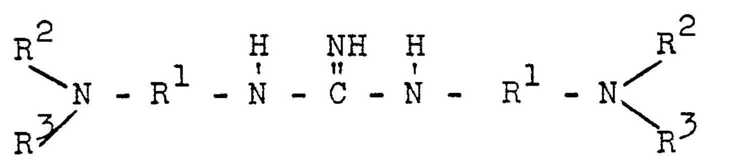 I,

worin die einzelnen Reste $R^1$, $R^2$ und $R^3$ jeweils einen aliphatischen Rest bedeuten, vorteilhaft erhält, wenn man Guanidiniumsalze der Formel

WB/BL

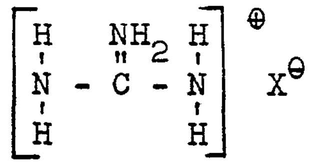 II,

worin X ein Säureanion bezeichnet, mit Diaminen der Formel

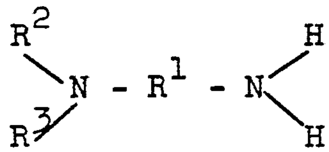 III,

worin $R^1$, $R^2$ und $R^3$ die vorgenannte Bedeutung besitzen, bei einer Temperatur von 140 bis 250°C umsetzt.

Weiterhin wurden die neuen 1,3-Bis-(dialkylaminoalkyl)-guanidine der Formel

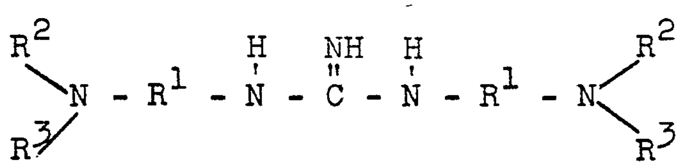 I,

worin die einzelnen Reste $R^1$, $R^2$ und $R^3$ jeweils einen aliphatischen Rest bedeuten, gefunden.

Die Umsetzung kann für den Fall der Verwendung von Guanidiniumhydrochlorid und 2-Dimethylaminoäthylamin durch das folgende Formelchema beschrieben werden:

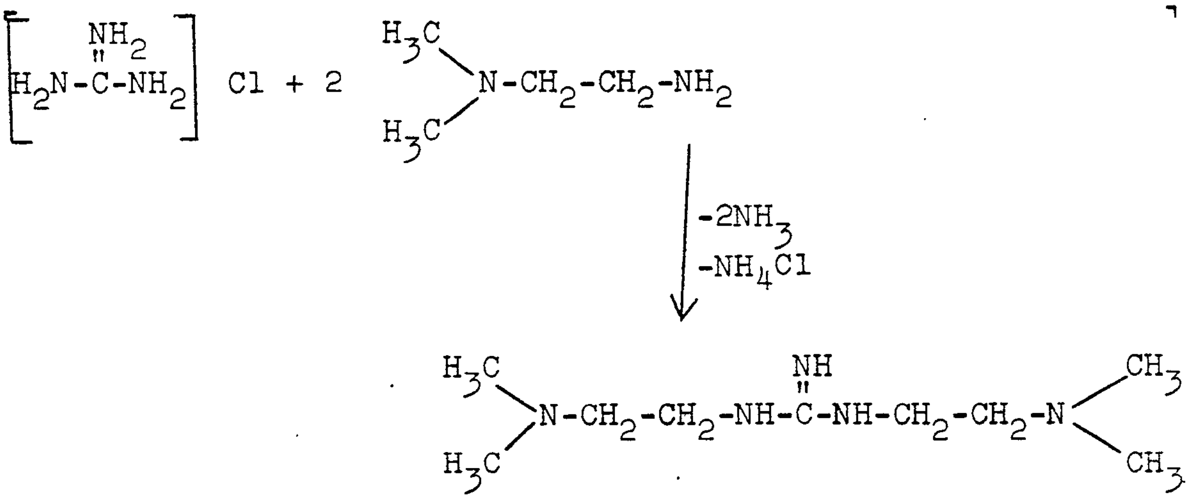


Im Hinblick auf den Stand der Technik liefert das Verfahren nach der Erfindung auf einfachem und wirtschaftlichem Wege die neuen 1,3-Bis-(dialkylaminoalkyl)-guanidine in guter Ausbeute und Reinheit. Alle diese vorteilhaften Ergebnisse des erfindungsgemäßen Verfahrens sind überraschend, da (Houben-Weyl, loc. cit., Seiten 187, 188; US-PS 2 230 965) bekannt ist, daß beim Zusammenschmelzen eines Guanidiniumsalzes mit einem Amin im wesentlichen monosubstituierte Guanidin-Derivate entstehen. Es ist bislang nicht bekannt, daß eine zweifache Umamidierung des Guanidins in wesentlichem Maße stattfinden kann.

Die Ausgangsstoffe II können mit den Ausgangsstoffen III in stöchiometrischer Menge oder im Überschuß, zweckmäßig in einer Menge von 2 bis 4, insbesondere 2,05 bis 2,5 Mol Ausgangsstoff III je Äquivalent Ausgangsstoff II umgesetzt werden. Bevorzugte Ausgangsstoffe II und III und dementsprechend bevorzugte Endstoffe I sind solche, in deren Formeln die einzelnen Reste $R^1$ jeweils einen Alkylenrest mit 2 bis 6 Kohlenstoffatomen bedeuten, die einzelnen Reste $R^2$ und $R^3$ jeweils einen Alkylrest mit 1 bis 6 Kohlenstoffatomen bezeichnen, wobei jeweils die Reste $R^2$ und $R^3$ untereinander gleich oder verschieden sein können, X ein Säureanion bezeichnet. Die vorgenannten Reste können noch durch

unter den Reaktionsbedingungen inerte Gruppen, z.B. Alkylgruppen oder Alkoxygruppen mit jeweils 1 bis 4 Kohlenstoffatomen, substituiert sein.

Es kommen z.B. folgende Diamine als Ausgangsstoffe III in Frage: 2-(N,N-Dimethylamino)-, 2-(N,N-Dipropylamino)-, 2-(N,N-Diäthylamino)-, 2-(N,N-Diisopropylamino)-, 2-(N,N-Dibutylamino)-, 2-(N,N-Diisobutylamino)-, 2-(N,N-Di-sek.-butylamino)-, 2-(N,N-Di-tert.-butylamino)-, 2-(N-Methyl-N-äthylamino)-äthylamin; entsprechend N,N-substituierte 3-Aminopropylamine, 4-Aminobutylamine, 5-Aminopentylamine, 6-Aminohexylamine, α- und ß-Aminoisopropylamine, α-, ß- und γ-Aminoisobutylamine, γ- oder ß-Amino-sek.-butylamine, ß-Amino-tert.-butylamine, 2-Aminopropylamine, 2-Aminobutylamine, 3-Aminobutylamine.

Die Umsetzung wird bei einer Temperatur von 140 bis 250°C, vorzugsweise 170 bis 200°C, drucklos oder unter Druck, kontinuierlich oder diskontinuierlich durchgeführt. In der Regel dient die Schmelze des Gemischs der Ausgangsstoffe II und III gleichzeitig als Reaktionsmedium der Umsetzung, so daß keine zusätzlichen Lösungsmittel benötigt werden.

Als Guanidiniumsalze II können Salze anorganischer oder organischer Säuren verwendet werden. Anstelle einbasischer Säuren können auch äquivalente Mengen mehrbasischer Säuren zur Anwendung gelangen. Von mehrbasischen, insbesondere zweibasischen Säuren kommen mono-, di- oder polybasische Salze, z.B. das Carbonat, Bicarbonat, Sulfat, Hydrogensulfat, Phosphat, Hydrogenphosphat, Dihydrogenphosphat, in Frage. Beispielsweise sind die Salze folgender Säuren geeignet: Salzsäure, Bromwasserstoffsäure, Jodwasserstoffsäure, Fluorwasserstoffsäure, Schwefelsäure, Phosphorsäure, schweflige Säure, Kohlensäure; Sulfonsäuren wie Methylsulfonsäure, Monomethylsulfat, Benzol- und p-Toluolsulfonsäure; Tetrafluorborsäure, Borsäure; aliphatische Carbonsäuren wie Oxalsäure, Ameisensäure, Cyanessigsäure, Essigsäure, Propionsäure, Buttersäure, Isobuttersäure, Caprylsäure, Trimethylessigsäure, Bernsteinsäure, Isovaleriansäure, Valeriansäure, Glutarsäure, Adipinsäure; cycloaliphatischen, araliphatischen, aromatischen Carbonsäuren wie Benzoesäure, 2,3-, 2,4-, 2,5-, 2,6-Dimethylbenzoesäure, Phenylpropionsäure, Cyclohexancarbonsäure, Phenylessigsäure, α- bzw. ß-Naphthoesäure, Phthalsäure, o-, m- bzw. p-Toluylsäure, o-, m- bzw. p-Nitrobenzoesäure, Isophthalsäure, Terephthalsäure. Bevorzugt sind die Hydrochloride, Hydrogensulfite und Sulfate.

Die Reaktion wird zweckmäßig wie folgt durchgeführt: Ein Gemisch von stöchiometrischen Mengen bis zu einem geringen Überschuß an Ausgangsstoff III in der Schmelze von Ausgangsstoff II wird bei vorgenannter Reaktionstemperatur während 2 bis 40, insbesondere 2 bis 15 Stunden umgesetzt, dann der Endstoff in üblicher Weise, z.B. durch Verrühren mit Natronlauge, Abtrennung der organischen Phase aus dem gebildeten 2-Phasengemisch und Destillation, isoliert. Zweckmäßig gibt man erst nach der Reaktion den vorgenannten Überschuß an Ausgangsstoff III als Verdünnungsmittel zu, um die Viskosität der organischen Phase zu verringern und so die Abtrennung von der wäßrigen Phase zu erleichtern.

Die nach dem Verfahren der Erfindung herstellbaren neuen 1,3-Bis-(dialkylaminoalkyl)-guanidine I sind wertvolle Ausgangsstoffe für die Herstellung von Hilfsmitteln auf dem Kunststoffgebiet, Farbstoffen, Pflanzenschutzmitteln und Pharmazeutika. Sie sind im allgemeinen hochviskose Flüssigkeiten und eignen sich z.B. als Katalysatoren zur Herstellung von Polyurethanen (siehe Beispiele 5 bis 7 und Vergleichsbeispiele 8 bis 10).

Als Katalysatoren werden zur Herstellung von Polyurethanen, insbesondere von Polyurethanschaumstoffen, tertiäre Amine verwendet. Nachteilig ist, daß tertiäre Amine, die keine aktiven Wasserstoffatome besitzen, z.B. Diazobicyclo-[2,2,2]-octan, N-Äthylmorpholin, Dimethylcyclohexylamin, Pentamethyldiäthylentriamin, Tetramethylbutylendiamin oder Bis-(dimethylaminoäthyl)-äther nicht in die Polyurethankunststoffe eingebaut werden, so daß sie im Laufe der Zeit auswandern können. Dies kann zu unerwünschten Belästigungen, wie Geruch oder Hautreizungen führen oder es kann der hydrolytische Abbau des fertigen Schaumstoffes beschleunigt werden. Eine andere Schwierigkeit liegt darin, daß nicht in jedem Fall die ablaufenden Reaktionen so beschleunigt werden, wie es ein optimales Reaktionsgeschehen hinsichtlich Verarbeitbarkeit der Ausgangsstoffe und Endeigenschaften der Polyurethane erfordert. Tertiäre Amine mit reaktiven Wasserstoffatomen, z.B. 2-N,N-Dialkylamino-äthanole, N-Alkyldiäthanolamine und Triäthanolamine zeigen aufgrund ihres Einbaus in das Polyurethan eine schnelle Abnahme der katalytischen Wirkung, so daß es zu einem unerwünscht verzögerten Reaktionsablauf oder gar -stillstand kommen kann. Hochmolekulare Katalysatoren, wie tertiäre Aminogruppen haltige Polyätherole, sind zwar geruchsfrei, weisen aber in der Regel eine zu geringe katalytische Aktivität auf.

Im Vergleich zu den bekannten Katalysatoren zeigen die Endstoffe I keinen der obengenannten Mängel in deutlichem Maße, insbesondere keine Geruchsbelästigung und Hautreizungen; begünstigen mögliche Nebenreaktionen, wie die Bildung von Allophanat-, Biuret-, Isocyanurat- und Carbodiimidgruppen, nicht und gewährleisten andererseits den synchronen Ablauf von Polyurethanbildung und gegebenenfalls Schäumvorgang.

Aus ihnen können in an sich bekannter Weise durch Umamidierung von Thioharnstoff bzw. Umsetzung mit Schwefelkohlenstoff Thioharnstoffe hergestellt werden. (Houben-Weyl, Methoden der Organischen Chemie, 4. Auflage, Band 9, Seiten 885 und 899, Georg Thieme Verlag, Stuttgart, 1955). Solche Thioharnstoffe haben die Formel

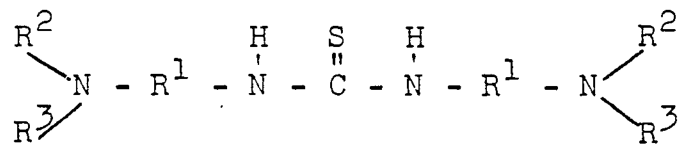 IV,

worin $R^1$, $R^2$ und $R^3$ die vorgenannten allgemeinen und bevorzugten Bedeutungen besitzen.
Unter den Stoffen I und IV sind solche der Formel

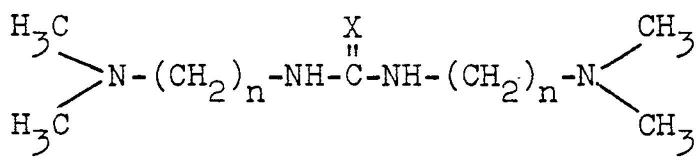 V,

in der X Schwefel oder eine =NH-Gruppe und n eine ganze Zahl von 2 bis 6 bedeuten, sowie deren Gemische besonders bevorzugt.

Die genannten Guanidine I und Thioharnstoffe IV besitzen schwach reaktive Wasserstoffatome und können daher in das Polyurethan eingebaut werden. Vorteilhaft ist hierbei, daß der Einbau des Katalysators nicht zu früh, sondern langsam bzw. erst bei höheren Temperaturen erfolgt, so daß seine katalytische Wirksamkeit während der gesamten Polyurethanbildung erhalten bleibt.

Als Beispiele für die erfindungsgemäß verwendbaren Guanidine I und Thioharnstoffe IV seien insbesondere genannt: N,N'-Bis-(dimethylamino-äthyl)-guanidin, N,N'-Bis-(dimethylamino-propyl)-guanidin, N,N'-Bis-(dimethylamino-butyl)-guanidin, N,N'-Bis-(dimethylamino-äthyl)-thioharnstoff, N,N'Bis-(dimethylamino-propyl)-thioharnstoff und N,N'-Bis-(dimethylamino-butyl)-thioharnstoff. Die Guanidine I und Thioharnstoffe IV können einzeln sowie in Form von Mischungen Anwendung finden. Vorzugsweise verwendet werden N,N'-Bis-(dimethylamino-propyl)-thioharnstoff, N,N'-Bis-(dimethylamino-butyl)-thioharnstoff und N,N'-Bis-(dimethylamino-propyl)-guanidin.

Zur Herstellung der Polyurethane werden in der Regel 0,01 bis 5 Gew.-%, vorzugsweise 0,5 bis 3 Gew.-% Guanidin und/oder Thioharnstoff, bezogen auf das Gesamtgewicht von Polyisocyanat und höhermolekularer Polyhydroxylverbindung, eingesetzt. Es können thermoplastische Elastomere und weichelastische, halbharte und harte Polyurethanschaumstoffe sowie Integralschaumstoffe hergestellt werden. Besonders geeignet und daher vorzugsweise verwendet werden die Guanidine und Thioharnstoffe als Katalysatoren zur Herstellung von Polyurethanweichschaumstoffen. Als verwendbare Ausgangsstoffe kommen organische Polyisocyanate, z.B. aliphatische, cycloaliphatische und vorzugsweise aromatische mehrwertige Isocyanate in Betracht. Im einzelnen seien beispielhaft genannt: Alkylendiisocyanate mit 2 bis 14 Kohlenstoffatomen im Alkylenrest, wie Äthylendiisocyanat, 1,4-Tetramethylendiisocyanat, 1,12-Dodecandiisocyanat und vorzugsweise 1,6-Hexamethylendiisocyanat; cycloaliphatische Di- und Polyisocyanate, wie Cyclobutan-1,3-diisocyanat, Cyclohexan-1,3- und -1,4-diisocyanat sowie beliebige Gemische dieser Isomeren, Hexahydrotoluylen-2,4- und -2,6-diisocyanat sowie die entsprechende Isomerengemische 2,2'-, 2,4'- und 4,4'-Dicyclohexylmethan-diisocyanat und die entsprechenden Isomerengemische, Polycyclohexyl-polymethylen-polyisocyanate, Gemische aus Dicyclohexylmethan-diisocyanaten und Polycyclohexyl-polymethylen-polyisocyanaten und vorzugsweise 1-Isocyanato-3,3,5-trimethyl-5-isocyanatomethyl-cyclohexan und vorzugsweise aromatische Di- und Polyisocyanate, wie 1,3- und 1,4-Phenylendiisocyanat, 2,2'-, 2,4'- und 4,4'-Diphenylmethan-diisocyanat sowie die entsprechenden Isomerengemische, 2,4- und 2,6-Toluylen-diisocyanat sowie die entsprechenden Isomerengemische 2,4,6-Triisocyanato-toluol, Triphenylmethan-4,4',4"-triisocyanat und Polyphenyl-polymethylen-polyisocyanate und vorzugsweise Gemische aus Di- und Polyphenyl-polymethylenpolyisocyanaten (Roh-MDI). Die genannten Di- und Polyisocyanate können einzeln oder in Form von Mischungen eingesetzt werden.

Häufig werden auch sogenannte modifizierte mehrwertige Isocyanate, d.h. Produkte die durch chemische Umsetzung obiger Di- und/oder Polyisocyanate erhalten werden, verwendet. Als modifizierte organische Di- und Polyisocyanate kommen beispielsweise in Betracht:
Carbodiimidgruppen aufweisende Polyisocyanate gemäß DT-PS 10 92 007, Allophanatgruppen aufweisende Polyisocyanate, wie sie z.B. in der britischen Patentschrift 994 890, den ausgelegten Unterlagen des belgischen Patents 761 626 und der NL-OS 71 02 524 beschrieben werden, Isocyanuratgruppen aufweisende Polyisocyanate wie sie z.B. in den DE-PS 10 22 789, 12 22 067 und 10 27 394 sowie den DE-OS 19 29 034 und 20 04 048 beschrieben werden, Urethangruppen aufweisende Polyisocyanate, wie sie z.B. in den ausgelegten Unterlagen des belgischen Patents 752 261 oder der US-PS 33 94 164 beschrieben werden, acylierte Harnstoffgruppen aufweisende Polyisocyanate z.B. gemäß DE-PS 12 30 778, Biuretgruppen aufweisende Polyisocyanate z.B. gemäß DE-PS 11 01 394 und GB-PS 889 050; durch Telomerisationsreaktionen hergestellte Polyisocyanate z.B. entsprechend den ausgelegten Unterlagen des belgischen Patents 723 640, Estergruppen aufweisende Polyisocyanate,

wie sie z.B. in der GB-PS 965 474 und 10 72 956, der US-PS 35 67 765 und der DE-PS 12 31 688 genannt werden.

Vorzugsweise kommen jedoch zur Anwendung: urethangruppenhaltige Polyisocyanate, Isocyanuratringe enthaltende Polyisocyanate und insbesonders Toluylen-diisocyanate, Diphenylmethan-diisocyanate, Mischungen aus Diphenylmethan-diisocyanaten und Polyphenyl-polymethylen-polyisocyanaten (Roh-MDI) und Gemische aus Toluylen-diisocyanaten und Roh-MDI.

Als weitere Ausgangsstoffe kommen höhermolekulare, organische Polyhydroxylverbindungen in Frage. Sie sind vorzugsweise übliche lineare und/oder verzweigte Polyesterole und insbesondere Polyätherole mit Molekulargewichten von 200 bis 8 000, vorzugsweise 800 bis 5 000, und insbesondere 1 800 bis 3 500. In Betracht kommen jedoch auch andere hydroxylgruppenhaltige Polymere mit den genannten Molekulargewichten, beispielsweise Polyesteramide, Polyacetale, wie Polyoxymethylene, und Polycarbonate, insbesondere solche, hergestellt aus Diphenylcarbonat und Hexandiol-1,6 durch Umesterung.
Die Polyesterole können beispielsweise aus Dicarbonsäuren, vorzugsweise aliphatischen Dicarbonsäuren, mit 2 bis 12, vorzugsweise 4 bis 8 Kohlenstoffatomen im Alkylenrest und mehrwertigen Alkoholen, vorzugsweise Diolen, hergestellt werden. Genannt seien beispielhaft aliphatische Dicarbonsäuren, wie Pimelinsäure, Korksäure, Azelainsäure, Sebacinsäure, Undecandisäure, Dodecandisäure und vorzugsweise Bernstein-, Glutar- und Adipinsäure, und aromatische Dicarbonsäuren, wie Phthalsäure und Terephthalsäure. Beispiele für zwei- und mehrwertige, insbesondere zwei- und dreiwertige Alkohole sind: Äthylenglykol, Diäthylenglykol, 1,2- bzw. 1,3-Propylenglykol, Dipropylenglykol, Decandiol-1,10, Glycerin, Trimethylolpropan und vorzugsweise Butandiol-1,4 und Hexandiol-1,6.

Sofern zur Herstellung der Polyesterole mehrwertige, insbesondere dreiwertige Alkohole mitverwendet werden, wird deren Gehalt zweckmäßigerweise so berechnet, daß die Funktionalität der erhaltenen Polyesterole maximal 2,5 ist.

Die Polyesterole besitzen Molekulargewichte von 500 bis 2 800, vorzugsweise von 1 000 bis 2 000, und Hydroxylzahlen von 40 bis 280, vorzugsweise von 50 bis 120.

Vorzugsweise als Polyhydroxylverbindungen verwendet werden jedoch Polyätherole, die nach bekannten Verfahren aus einem oder mehreren Alkylenoxiden mit 2 bis 4 Kohlenstoffatomen im Alkylenrest und einem Startermolekül, das 2 bis 8, vorzugsweise 2 bis 4 aktive Wasserstoffatome enthält, hergestellt werden.

Geeignete Alkylenoxide sind beispielsweise Tetrahydrofuran, 1,3-Propylenoxid, 1,2- bzw. 2,3-Butylenoxid, Styroloxid und vorzugsweise Äthylenoxid und 1,2-Propylenoxid. Die Alkylenoxide können einzeln, alternierend nacheinander oder als Mischungen verwendet werden. Als Startermoleküle kommen beispielsweise in Betracht:
Wasser, organische Dicarbonsäure, wie Bernsteinsäure, Adipinsäure, Phthalsäure und Terephthalsäure, aliphatische und aromatische, gegebenenfalls N-Mono-, N,N- und N,N'-dialkylsubstituierte Diamine mit 1 bis 4 Kohlenstoffatomen im Alkylrest, wie gegebenenfalls mono- und dialkylsubstituiertes Äthylendiamin, Diäthylentriamin, Triäthylentetramin, 1,3-Propylendiamin, 1,3- bzw. 1,4-Butylendiamin, 1,2-, 1,3-, 1,4-, 1,5- und 1,6-Hexamethylendiamin, Phenylendiamine, 2,4- und 2,6-Toluylendiamin und 4,4'-, 2,4'-, 2,2'-Diamino-diphenylmethan; Monoamine, wie Methylamin, Äthylamin, Isopropylamin, Butylamin, Benzylamin, Anilin, die Toluidine und Naphthylamine. Von den Verbindungen

der erwähnten Gruppe sind besonders interessant N,N,N',N'-Tetrakis-(2-hydroxyäthyl)-äthylendiamin, N,N,N',N'-Tetrakis-(2-hydroxypropyl)-äthylendiamin, N,N,N',N'',N''-Pentakis-(2-hydroxypropyl-)äthylentriamin, Phenyldiisopropanolamin und höhere Alkylenoxidaddukte von Anilin.

Als Startermoleküle kommen ferner in Betracht Alkanolamine, wie Äthanolamin, Diäthanolamin, N-Methyl- und N-Äthyl-diäthanolamin, N-Methyl- und N-Äthyl-diäthanolamin und Triäthanolamin, Hydrazin und Hydrazide. Vorzugsweise verwendet werden mehrwertige, insbesondere zwei- und/oder dreiwertige Alkohole, wie Äthylenglykol, Propylenglykol-1,2 und -1,3, Diäthylenglykol, Dipropylenglykol, Butylenglykol-1,4, Hexamethylenglykol-1,6, Glycerin, Trimethylol-propan und Pentaerythrit.

Andere anwendbare Polyhydroxylverbindungen sind die nicht reduzierenden Zucker, die nicht-reduzierenden Zuckerderivate und bevorzugt deren Alkylenoxid-Addukte, worin die Alkylenoxide 2 bis 4 Kohlenstoffatome haben. Verwendbare nicht-reduzierende Zucker und Zuckerderivate sind z.B. Saccarose, Alkylglykoside, wie Methylglykosid und Äthylenglukosid, ferner Glykolglykoside, wie Äthylenglykolglykosid, Propylenglukosid, Glyzeringlykosid und 1,2,6-Hexantriolglukosid.

In Betracht kommen ferner Polyhydroxylverbindungen auf Basis von Polyphenolen und vorzugsweise deren Alkylenoxid-Addukte, in denen die Alkylenoxide 2 bis 4 Kohlenstoffatome besitzen. Anwendbare Polyphenole sind z.B. Bisphenol A, Bisphenol F, Kondensationsprodukte aus Phenol und Formaldehyd, besonders die Novolake, Kondensationsprodukte aus verschiedenen Phenolverbindungen und Acrolein, wobei die einfachsten Substanzen dieser Gruppe die 1,1,3-Tris-(hydroxyphenyl)-propane sind, Kondensationsprodukte verschiedener Phenolverbindungen mit Glyoxal, Glutaraldehyd und anderen Dialdehyden, wobei die einfachsten Substanzen dieser Gruppe die 1,1,2,2-Tetrakis-(hydroxylphenyl)-äthane sind.

Eine weitere verwendbare Gruppe von Polyhydroxylverbindungen sind die Alkylenoxid-Addukte, vorzugsweise die Äthylenoxid-, 1,2-Epoxypropan-, Epoxybutan- und deren Mischungen, -Addukte von Kondensationsprodukten aus aromatischem Amin, Phenol und Aldehyd. Die Kondensationsprodukte erhält man durch Kondensieren eines aromatischen Amins, z.B. Anilin oder Toluidin, eines Phenols z.B. Phenol oder Kresol und eines Aldehyds, vorzugsweise Formaldehyd bei erhöhten Temperaturen, z.B. im Bereich von 60 bis 180°C. Das Kondensationsprodukt wird dann isoliert und unter Bildung der Polyole mit einem Alkylenoxid umgesetzt. Besonders erwähnenswert sind die Propylenoxid- und Propylen-Äthylenoxid--Addukte von Anilin/Phenol/Formaldehyd-Kondensationsprodukten.

Die Alkylenoxid-Addukte von Phosphor- und Polyphosphorsäuren sind eine weitere verwendbare Gruppe von Polyhydroxylverbindungen. Bevorzugte Alkylenoxide sind Äthylenoxid, 1,2-Epoxypropan, die Epoxybutane und 3-Chlor-1,2-Epoxypropan. Als Phosphorsäuren sind Phosphorsäure, phosphorige Säure, die Polyphosphorsäuren, wie Tripolyphosphorsäure, und die Polymetaphosphorsäuren günstig.

Bevorzugt verwendet werden Polyätherole mit Molekulargewichten von 400 bis 10 000, vorzugsweise von 400 bis 6 000, und Hydroxylzahlen von 20 bis 400, vorzugsweise von 25 bis 150, die sowohl Äthylenoxid als auch 1,2-Propylenoxideinheiten in der Oxyalkylenkette enthalten, wobei diese entweder statistisch oder blockweise in der Oxyalkylenkette angeordnet sein können. Insbesondere eingesetzt werden Polyätherole, die vorzugsweise primäre Hydroxylgruppen besitzen. Die verwendeten Polyhydroxylverbindungen können eine Hydroxylzahl aufweisen, die innerhalb eines breiten Bereiches variieren kann.

Welche höhermolekulare, organische Polyhydroxylverbindung jeweils verwendet wird, hängt von der Endverwendung des daraus herzustellenden Polyurethans ab.

Die Polyurethane, vorzugsweise Polyurethanschaumstoffe, werden aus den obengenannten organischen Polyisocyanaten und höhermolekularen organischen Polyhydroxylverbindungen in Gegenwart der erfindungsgemäß verwendbaren Guanidine I und/oder Thioharnstoffe IV als Katalysatoren sowie gegebenenfalls Cokatalysatoren, Treibmitteln, Kettenverlängerungsmitteln, Hilfs- und Zusatzstoffen hergestellt.

Geeignete Cokatalysatoren, die mit den erfindungsgemäß verwendbaren Guanidinen und/oder Thioharnstoffen zu Katalysatorkombinationen vereinigt werden, sind organische Zinnverbindungen. Beispielhaft genannt seien Zinn(II)-salze von Carbonsäuren, wie Zinn(II)-acetat, Zinn(II)-octoat, Zinn(II)-äthylhexoat und Zinn(II)-laurat und die Zinn(IV)-Verbindungen z.B. Dibutylzinnoxid, Dibutylzinndichlorid, Dibutylzinndiacetat, Dibutylzinnmaleat, Dioctylzinndiacetat und vorzugsweise Dibutylzinndilaurat. Die genannten Zinnverbindungen können selbstverständlich auch als Gemische mit den erfindungsgemäß verwendbaren Guanidinen und Thioharnstoffen kombiniert werden. Sofern zur Herstellung der Polyurethane Katalysatorkombinationen eingesetzt werden, bestehen diese vorteilhafterweise aus 0,01 bis 5 Gew.-%, vorzugsweise 0,5 bis 3 Gew.-% Guanidin und/oder Thioharnstoff und 0,01 bis 2 Gew.-%, vorzugsweise 0,1 bis 1 Gew.-% einer organischen Zinnverbindung.

Ein geeignetes Treibmittel ist Kohlendioxid, das aus Wasser und Polyisocyanaten gebildet wird. Die Wassermengen, die zweckmäßigerweise verwendet werden können, betragen 0,1 bis 2 %, bezogen auf das Gewicht an Polyisocyanat. Gegebenenfalls können auch größere Wassermengen verwendet werden, jedoch vorzugsweise dann nicht, wenn die thermische Stabilität oder die Wärmeisolationseigenschaften der erhaltenen Schaumstoffe von besonderer Bedeutung sind.

Andere verwendbare Treibmittel sind niedrig siedende Flüssigkeiten, die unter dem Einfluß der exothermen Polyadditionsreaktion verdampfen. Geeignet sind Flüssigkeiten, welche gegenüber dem organischen Polyisocyanat inert sind und Siedepunkte unter 100°C aufweisen. Beispiele derartiger, vorzugsweise verwendeter Flüssigkeiten sind halogenierte Kohlenwasserstoffe, wie Methylenchlorid, Trichlorfluormethan, Dichlordifluormethan, Dichlormonofluormethan, Dichlortetrafluoräthan und 1,1,2-Trichlor-1,2,2-trifluoräthan. Auch Gemische dieser niedrigsiedenden Flüssigkeiten untereinander und/oder mit anderen substituierten oder unsubstituierten Kohlenwasserstoffen können verwendet werden.

Die zweckmäßigste Menge an niedrigsiedender Flüssigkeit zur Herstellung von Polyurethanschaumstoffen hängt ab von der Schaumdichte, die man erreichen will sowie gegebenenfalls von der Mitverwendung von Wasser. Im allgemeinen liefern Mengen von 5 bis 40 Gewichtsprozent, bezogen auf 100 Gewichtsteile organisches Polyisocyanat, zufriedenstellende Ergebnisse.

Während man normale weiche oder halbharte Schaumtypen bevorzugt mit Wasser treibt, benutzt man zur Herstellung von

0033879

Integralschaumstoffen, d.h. Schäumen mit einer kompakten Außenhaut, gewöhnlich physikalische Treibmittel.

Gegebenenfalls ist es auch zweckmäßig zur Herstellung der Polyurethane neben den höhermolekularen Polyhydroxylverbindungen zusätzlich Kettenverlängerungsmittel zu verwenden. Die Kettenverlängerungsmittel besitzen Molekulargewichte kleiner als 2 000, vorzugsweise von 30 bis 600, und weisen vorzugsweise zwei aktive Wasserstoffatome auf. In Betracht kommen beispielsweise aliphatische und/oder aromatische Diole mit 2 bis 14, vorzugsweise 4 bis 10, Kohlenstoffatomen, wie Äthylenglykol, Propandiol, Decandiol-1,10 und vorzugsweise Butandiol-1,4, Hexandiol-1,6 und Bis-(2-hydroxyäthyl)-hydrochinon, Diamine, wie Äthylendiamin und 4,4'-Diaminodiphenylmethan, Äthanolamine, wie Triäthanolamin und Polyhdroxylverbindungen, wie Glycerin, Trimethylpropan und niedermolekulare hydroxylgruppenhaltige Polyalkylenoxide aus den vorgenannten Ausgangsstoffen.

Der Mischung können auch noch Hilfs- und Zusatzstoffe einverleibt werden. Genannt seien beispielsweise oberflächenaktive Schaumstabilisatoren, Hydrolysenschutzmittel, Porenregler, fungistatisch und bakteriostatisch wirkende Substanzen, Farbstoffe, Pigmente, Flammschutzmittel und Verstärkungsmittel.

In Betracht kommen beispielsweise oberflächenaktive Substanzen, welche zur Unterstützung der Homogenisierung der Ausgangsstoffe dienen und gegebenenfalls auch geeignet sind, die Zellstruktur der Schaumstoffe zu regulieren. Genannt seien beispielhaft Siloxan-Oxalkylen-Mischpolymerisate und andere Organopolysiloxane, oxäthylierte Alkylphenole, oxäthylierte Fettalkohole, Paraffinöle, Rizinusöl- bzw. Rizinolsäureester und Türkischrotöl, die in Mengen von 0,2 bis 6 Gewichtsteilen pro 100 Gewichtsteile Polyisocyanat angewandt werden.

Geeignete Flammschutzmittel sind beispielsweise Trikresylphosphat, Tris-2-chloräthylphosphat, Tris-chlorpropylphosphat und Tris-2,3-dibrompropylphosphat.

Außer den bereits genannten halogensubstituierten Phosphaten können auch organische Flammschutzmittel, wie Antimontrioxid, Arsenoxid, Ammoniumphosphat und Calciumsulfat zum Flammfestmachen der Polyurethanschaumstoffe verwendet werden. Im allgemeinen hat es sich als vorteilhaft erwiesen, 5 bis 50 Gewichtsteile, vorzugsweise 5 bis 25 Gewichtsteile der genannten Flammschutzmittel für jeweils 100 Gewichtsteile an organischem Polyisocyanat zu verwenden.

Als Verstärkungsmittel seien beispielsweise organische Polymer-Polyol-Gemische und anorganische Füllstoffe genannt. Die organische Polymer-Polyol-Gemische können hergestellt werden durch Mischen von wässerigen Homo- und/oder Copolymerdispersionen mit Polyolen und anschließendes Abtrennen von Wasser oder vorzugsweise durch in situ Pfropfpolymerisation von Styrol und insbesonders Styrol-Acrylnitrilmischungen in Polyolen. Als anorganische Verstärkungsmittel seien beispielsweise Lösungen von Alkalisalzen anorganischer Säuren, beispielsweise wässerige Kaliumfluorid-, Kaliumchlorid- und Natriumchloridlösungen genannt. Die Verstärkungsmittel werden üblicherweise in Mengen von 2 bis 20 Gew.-%, vorzugsweise 5 bis 13 Gew.-%, bezogen auf das Gesamtgewicht aus Polyisocyanat und Polyhydroxylverbindung verwendet.

Nähere Angaben über die obengenannten anderen üblichen Hilfs- und Zusatzstoffe sind der Fachliteratur, beispielsweise der Monographie von J.H. Saunders und K.C. Frisch "High Polymers" Band XVI, Polyurethanes, Teil 1 und 2, Verlag Interscience Publishers 1962 bzw. 1964, zu entnehmen.

Zur Herstellung der Polyurethane werden die organischen Polyisocyanate und Polyhydroxylverbindungen bzw. Mischungen aus Polyhydroxylverbindungen und Kettenverlängerungsmitteln in solchen Mengen zur Umsetzung gebracht, daß das Verhältnis von NCO : OH-Gruppen 1 : 0,8 bis 1,2, vorzugsweise ungefähr 1 : 1 beträgt.

Die Polyurethane, vorzugsweise Polyurethanschaumstoffe, werden nach dem Prepolymer- und vorzugsweise nach dem one shot-Verfahren hergestellt. Bei Verwendung einer Mischkammer mit mehreren Zulaufdüsen können die Ausgangskomponenten einzeln zugeführt und in der Mischkammer intensiv vermischt werden. Als besonders vorteilhaft hat es sich erwiesen, nach dem Zweikomponenten-Verfahren zu arbeiten und die Mischung aus höhermolekularen Polyhydroxylverbindung, Katalysator und gegebenenfalls Kettenverlängerungsmittel, Treibmittel, Hilfs- und Zusatzstoffen zu der Komponente A zu vereinigen und als Komponente B die organischen Polyisocyanate zu verwenden. Vorteilhaft ist hierbei beispielsweise, daß die Komponenten A und B getrennt gelagert und raumsparend transportiert werden können.
Zur Herstellung der Polyurethane werden die beschriebenen Ausgangsstoffe in den genannten Mengenverhältnissen intensiv bei Temperaturen von 20° bis 60°C, vorzugsweise 25° bis 45°C gemischt.

Die reaktionsfähigen Mischungen für Polyurethan-Elastomere werden in offene Formen oder auf Bleche gegossen und reagieren dort aus. Zur Vervollständigung der Umsetzung werden die erstarrten Produkte bei 60° bis 150°C, vorzugsweise 80° bis 120°C 2 bis 48 Stunden, vorzugsweise 12 bis 24 Stunden getempert. Die erhaltenen Elastomere besitzen Dichten von 1,1 bis 0,8 g/cm$^3$, Shore A Härten von 50 bis 100 und eignen sich beispielsweise zur Herstellung von Rollen, Puffern, Stoßfängern und Riemen.

Schaumfähige Reaktionsmischungen läßt man in offenen oder geschlossenen Formen zu Polyurethanschaumstoffe aufschäumen.

Je nach Art der hergestellten Polyurethanschaumstoffe besitzen die Produkte Dichten von 30 bis 300 kg/m$^3$ für weichelastische Schaumstoffe, von 60 bis 300 kg/m$^3$ für halbharte Schaumstoffe und von 10 bis 300 kg/m$^3$ für harte Schaumstoffe sowie durchschnittliche Dichten von 100 bis 800 kg/m$^3$ für Integralschaumstoffe.

Die erhaltenen Polyurethanschaumstoffe werden in den üblichen Verwendungsbereichen von Polyurethanen, wie Möbel- und Polsterschaum, Isolierschaum sowie zur Herstellung von Fertigteilen im Automobilbereich, wie Armlehnen, Stoßstangen, Armaturentafeln usw. eingesetzt.

Die in den folgenden Beispielen genannten Teile bedeuten Gewichtsteile.

Beispiel 1

In einer Rührapparatur werden unter Stickstoff 955 Teile Guanidiniumhydrochlorid bei 190°C geschmolzen. Unter Rühren gibt man 1 848 Teile 2-Dimethylaminoäthylamin während 13 Stunden bei 185°C zu. Anschließend läßt man das Gemisch noch 1/2 Stunde bei 185°C nachrühren. Danach gibt man innerhalb von 15 Minuten 1 000 Teile 2-Dimethylaminoäthylamin zu. Nach Abkühlen auf Raumtemperatur wird das Gemisch mit 4 000 Teilen 30-gewichtsprozentiger Natronlauge während 10 Minuten gerührt. Die hierbei gebildete obere Phase wird abgetrennt und bei 160°C (25 mbar) über einen Dünnschichtverdampfer destilliert. Man erhält als Rückstand 1 959 Teile (97 % der Theorie) 1,3-Bis-(2'-dimethylaminoäthyl)-guanidin als hoch-

viskose Flüssigkeit.

| | | | |
|---|---|---|---|
| $^1$H-NMR: | Multiplett | $\delta$ = 6,5 - 7,2 ppm | (3 H ; NH) |
| | Triplett | $\delta$ = 3,3 ppm | (4 H) |
| | Triplett | $\delta$ = 2,5 ppm | (4 H) |
| | Singulett | $\delta$ = 2,3 ppm | (12 H) |

Beispiel 2

In einer Rührapparatur werden unter Stickstoff 191 Teile Guanidiniumhydrochlorid bei 195°C geschmolzen. Unter Rühren gibt man während 10 Stunden 410 Teile 3-Dimethylaminopropylamin bei 187°C zu. Anschließend läßt man das Gemisch noch 2 Stunden bei 180°C nachrühren. Danach gibt man innerhalb von 10 Minuten weitere 200 Teile 3-Dimethylaminopropylamin zu. Man läßt das Reaktionsgemisch auf 30°C abkühlen und rührt es 10 Minuten mit 1 000 Teilen 30-gewichtsprozentiger Natronlauge. Die hierbei gebildete obere Phase wird abgetrennt und bei 150°C (15 mbar) in einem Rotationsverdampfer vom überschüssigen 3-Dimethylaminopropylamin befreit. Man erhält 437 Teile (95 % der Theorie) 1,3-Bis-(3'-dimethylamino-n-propyl)-guanidin als hochviskose Flüssigkeit.

| | | | |
|---|---|---|---|
| $^1$H-NMR: | Multiplett | $\delta$ = 7,1 - 7,8 ppm | (3 H; NH) |
| | Triplett | $\delta$ = 3,35 ppm | (4 H) |
| | Triplett | $\delta$ = 2,35 ppm | (4 H) |
| | Singulett | $\delta$ = 2,3 ppm | (12 H) |
| | Multiplett | $\delta$ = 1,8 ppm | (4 H) |

Beispiel 3

In einer Rührapparatur werden unter Stickstoff 95,5 Teile Guanidiniumhydrochlorid bei 190°C geschmolzen. Unter Rühren gibt man während 25 Stunden 240 Teile 4-Dimethylamino-n-butylamin bei 180°C zu. Anschließend läßt man das

Gemisch noch eine Stunde bei 180°C nachrühren, gibt dann weitere 100 Teile 4-Dimethylaminobutylamin zu und läßt dann auf 22°C abkühlen. Dann verrührt man das Gemisch mit 500 Teilen 30-gewichtsprozentiger Natronlauge. Die hierbei gebildete obere Phase wird abgetrennt und bei 170°C (15 mbar) in einem Rotationsverdampfer vom überschüssigen 4-Dimethylaminobutylamin befreit. Man erhält 253 Teile (98 % der Theorie) 1,3-Bis-(4'-dimethylamino-n-butyl)-guanidin als hochviskose Flüssigkeit.

$^1H$ - NMR: Multiplett $\delta$ = 6,8 - 7,5 ppm (3 H; NH)
Triplett $\delta$ = 2,8 ppm (4 H)
Triplett $\delta$ = 2,4 ppm (4 H)
Singulett $\delta$ = 2,2 ppm (12 H)
Multiplett $\delta$ = 1,5 ppm (8 H)

Beispiel 4

In einer Rührapparatur werden unter Stickstoff 95,5 Teile Guanidiniumhydrochlorid geschmolzen. Unter Rühren gibt man 500 Teile 3-Di-n-hexylaminopropylamin innerhalb von 12 Stunden bei 190°C zu und läßt anschließend das Gemisch noch eine Stunde bei 190°C nachrühren. Anschließend gibt man innerhalb von 5 Minuten weitere 200 Teile 3-Di-n-hexalaminopropylamin zu und verrührt den Kolbeninhalt mit 500 Teilen 30-gewichtsprozentiger Natronlauge. Die hierbei gebildete obere Phase wird abgetrennt und bei 180°C (0,1 mbar) im Rotationsverdampfer von überschüssigem Amin befreit. Man erhält 467 Teile (93 % der Theorie) 1,3-Bis-(3'-di-n-hexylaminopropyl)-guanidin als hochviskose Flüssigkeit.

$^1H$ - NMR: Multiplett $\delta$ = 6,4 - 7,0 ppm (3 H; NH)
Triplett $\delta$ = 3,3 ppm (4 H)
Triplett $\delta$ = 2,4 ppm (4 H)
Triplett $\delta$ = 2,3 ppm (8 H)
Multiplett $\delta$ = 1,1 - 1,7 ppm (36 H)
Triplett $\delta$ = 0,9 ppm (12 H)

Anwendungsbeispiele 5 bis 7 und Vergleichsbeispiele 8 bis 10

Komponente A ist eine Mischung aus 95 Teilen eines Polyätherols mit einer Hydroxylzahl von 25 und einem Molekulargewicht von 6 500, 1,5 Teilen Glycerin, 1,6 Teilen Wasser und 0,75 Teilen Katalysator.

Die Komponente B ist ein unter der Bezeichnung (R)Lupranat M20 im Handel erhältliches Polyphenylpolymethylenpolyisocyanat mit einem NCO-Gehalt von 32 Gewichtsprozent, das durch Anilin-Formaldehyd-Kondensation und anschließende Phosgenierung erhalten wird.

Komponente A und Komponente B werden binnen 10 Sekunden mit einem Rührer mit einer Drehzahl von 1 000 Umdrehungen pro Minute intensiv gemischt. Die homogene Mischung wird in eine quaderförmige Blockform in den Abmessungen 10 cm x 10 cm x 20 cm gegossen und die Schaumzeiten des gebildeten Halbhartschaumstoffes mit einem Apparat der Fluidyne Corp. (J. of Cellular Plastics, Mai - Juni 69, Seiten 159 bis 179, insbesondere Seite 171) aufgenommen. Die verwendeten Katalysatoren, das Mischungsverhältnis der Komponente B mit der Komponente A und die gemessenen Topfzeiten $t_1$ und Steigzeiten $t_2$ sind in Tabelle 1 zusammengefaßt. Die mit den erfindungsgemäßen Katalysatoren hergestellten halbharten Polyurethanschaumstoffe besitzen Raumgewichte von 170 $kg/m^3$ und eine Stauchhärte von 34 KPa (DIN 53 421) bei 20 % Stauchung.

Tabelle 1

| Beispiel | Katalysator | Molekulargewicht | Topfzeit $t_1$ (sec) | Steigzeit $t_2$ (sec) | Mischungsverhältnis Komponente B : A (Teile) |
|---|---|---|---|---|---|
| 5 | 1,3-Bis-(2'-dimethylamino-äthyl)-guanidin | 173 | 48 | 350 | 49 : 98,85 |
| 6 | 1,3-Bis-(3'-dimethylamino-n-propyl)-guanidin | 201 | 34 | 218 | 49 : 98,85 |
| 7 | 1,3-Bis-(4'-dimethylamino-n-butyl)-guanidin | 229 | 25 | 190 | 49 : 98,85 |
| 8 (Vergleich) | Diazabicyclo-2,2,2-octan (33 Gew.% in Dipropylenglykol) | 112 | 60 | 240 | 49 : 98,85 |
| 9 (Vergleich) | Dimethyläthanolamin | 89 | 45 | 270 | 49 : 98,85 |
| 10 (Vergleich) | Stickstoff-haltiges Polyol Hydroxylzahl = 35 | 4 800 | 60 | 500 | 79 : 100 |

## Patentansprüche

1. Verfahren zur Herstellung von 1,3-Bis-(dialkylaminoalkyl)-guanidinen der Formel

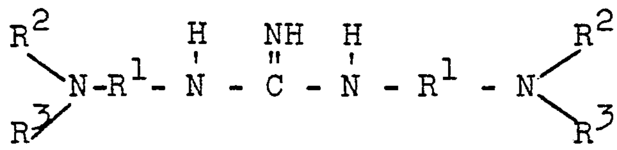

I,

worin die einzelnen Reste $R^1$, $R^2$ und $R^3$ jeweils einen aliphatischen Rest bedeuten, <u>dadurch gekennzeichnet</u>, daß man Guanidiniumsalze der Formel

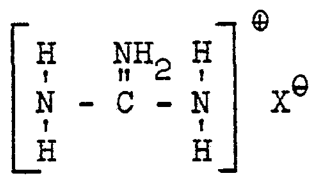

II,

worin X ein Säureanion bezeichnet, mit Diaminen der Formel

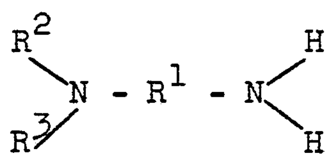

III,

worin $R^1$, $R^2$ und $R^3$ die vorgenannte Bedeutung besitzen, bei einer Temperatur von 140 bis 250°C umsetzt.

2. 1,3-Bis-(dialkylaminoalkyl)-guanidine der Formel

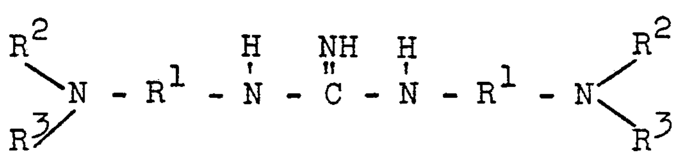

I,

worin die einzelnen Reste $R^1$, $R^2$ und $R^3$ jeweils einen aliphatischen Rest bedeuten.